# EUROPEAN PATENT APPLICATION

(11) **EP 1 205 195 A1**
(43) Date of publication of application: **15.05.2002**
(21) Application number: 01870123.5
(22) Date of filing: 08.06.2001
(51) Int. Cl.: A61L 9/03, A01M 1/20

(54) **Controllably activatable self- heating devices for dispensing volatile materials**

(30) Priority: 07.11.2000 EP 00870261; 07.11.2000 EP 00870262; 07.11.2000 EP 00870263
(71) Applicant: THE PROCTER & GAMBLE COMPANY, Cincinnati, Ohio 45202 (US)
(72) Inventor: Blenkiron, Peter, Egham, Surrey, TW20 8XB (GB); Burrowes, Lee, Woking, Surrey GU21 4PR (GB); Curtis, Terence Graham, High Wycombe, Bucks, HP11 1JN (GB); Metzger-Groom, Sabine Ursula, Morpeth, Northumberland, NE61 2DJ (GB); Addison, Michael Crombie, Newcastle upon Tyne, NE12 7YW (GB)
(74) Representative: Canonici, Jean-Jacques

(57) **Abstract**

Disclosed are devices for dispensing volatile materials such as fragrances into the atmosphere surrounding each such device. Release of volatile materials from a volatilization zone in such a device is promoted by the generation of heat via the non-combustive oxidation reaction of thermogenic materials held within a heating zone in the device. Heat is produced when these thermogenic materials are contacted with air. The devices are also provided with means for controlling contact of the thermogenic materials with air. Such means comprise both means for initiating contact of thermogenic materials with air to activate the device as well as additional means for thereafter increasing, decreasing or terminating contact of air with the thermogenic materials. The air contract control means associated with the devices herein thus serves to control and regulate the amount of heat produced within the device.

## Description

### Technical Field/Field of the Invention

The present invention relates to self-heating devices for dispensing volatile materials such as fragrances into the atmosphere surrounding the device.

### Background of the Invention

Devices for providing and dispensing volatile materials are well-known in the art. Such devices can include room freshners or fragrancing articles, vaporizers for humidification or dispensing and dispersal of therapeutic vapors, incense sticks, fragrancing or insect-repelling candles, and the like. Such devices generally involve utilization of some source of heat which promotes the volatilization of the materials to be dispensed and, of course, some source of the volatile or volatilizable materials themselves.

Some fragrancing devices, such as candles and incense sticks, involve use of an open flame or an active combustion reaction to provide the source of heat which promotes volatilization. Besides the obvious drawbacks of the hazards of using such types of heat sources, arrangements which involve flames or combustion are not especially portable and cannot be used, for example, in automobiles or around flammable materials.

Other types of dispensing devices for volatiles use electrical energy as a heat source. Plug-in room air-fresheners, for example, are commercially marketed under the tradenames *Ambi-Pur* and *Glade.* The amount of electrical energy required to operate devices of this type renders such devices relatively non-portable.

Some articles and devices for emitting or dispensing volatile materials employ non-combustive thermogenic materials as a heat source useful for promoting volatilization of the ingredients to be emitted or dispensed. Villamarin; PCT Patent Application WO 91/07996; Published June 13, 1991, for example, discloses a self-contained fragrance delivery package, comprising a fluent volatilizable fragrance material and an air-activatable chemical heat source. Disclosed configurations include a layered pad with covered perforations which can be removed to expose the heat source to air and activate the pad. Other dispensing devices using a non-combustive chemical heat source are disclosed in Takei; U.S. Patent 4,330,506; Issued May 18, 1982; Koiso et al; U.S. Patent 4,516,564; Issued May 14, 1985 and Arao; Japanese Patent Application 63175771; Published January 26, 1990.

Such devices in general are not necessarily readily consumer activatable and the generation of thermal energy within such devices may not be easily or readily controlled. Given this situation, there is a continuing need to provide dispensing devices in forms which are safe, portable, readily consumer activatable and controllable so as to be especially effective at dispensing the desired volatile materials in suitable amounts, at the desired time and over an acceptably prolonged period of time.

### Summary of the Invention

The present invention is directed to dispensing devices suitable for providing controlled discharge of volatilized active materials into the atmosphere surrounding the device. The invention further relates to the use of such a device for discharging and emitting volatilized active materials such as fragrances.

In its broadest sense, the devices herein comprise at least one volatilization zone containing volatilizable active material and at least one heating zone containing air-activatable thermogenic materials. The volatilization zone has a discharge end which is open or openable to the ambient atmosphere and is juxtaposed with and in thermodynamic communication with the heating zone. The thermogenic materials in the heating zone are capable of producing thermal energy via a non-combustive reaction when contacted with air. The thermal energy so produced must be transferable to the volatilization zone to thereby volatilize the volatilizable active material(s) therein and to further thereby permit and promote discharge of the volatilized materials through the discharge end of the volatilization zone and into the surrounding atmosphere.

Finally the devices herein essentially comprise means for controlling contact of the thermogenic materials in the heating zone with air. Such control of air contact must be suitable for permitting initiation of the generation of thermal energy from the thermogenic materials and must also be suitable for thereafter either increasing, decreasing and/or terminating generation of thermal energy from such materials.

In a preferred configuration, the dispensing devices herein comprise an insert, which itself comprises the volatilization and heating zones, and a holder for this insert. One zone of the insert is a substantially rigid volatilization zone which contains volatile or volatilizable materials. The other zone of the insert is a heating zone which contains air-activatable thermogenic materials. This volatilization zone and the heating zone are juxtaposed and in thermodynamic communication with each other within the insert.

In preferred devices, both the volatilization zone and the heating zone of the insert are sealed from the atmosphere surrounding the insert by rupturable sealing means such as, for example, aluminum foil. Such sealing means can be ruptured to expose both zones to the atmosphere. Such exposure activates the thermogenic materials within the heating zone, thereby releasing volatilizing heat, and this also releases volatilized materials from the volatilization zone into the atmosphere. Once activated, further means can then be provided to alter and control the contact of air with the thermogenic materials in the heating zone, thereby regulating the heat produced.

### Brief Description of the Drawings

Figure 1 is a view in perspective of a fragrance dispensing device prior to the activation of the device.
Figure 2 is a view in perspective of a fragrance dispensing device after the device has been activated.
Figure 3 is a section view of the pre-activated device of Figure 1.
Figure 4 is an exploded view of the insert element of a device according to the present invention.
Figure 5 is a cut-away view of a fragrance device positioned within an airtight container which can serve to deactivate the device.

### Detailed Description of the Invention

The dispensing devices of the present invention comprise both volatilization and heating zones. These zones are preferably found within an insert which is carried and held within a two-part holder. The devices can also comprise various forms of air contact control means. Elements of various dispensing device configurations herein are described in detail as follows:

### A) Insert

An insert is the component of a preferred dispensing device which holds both the volatilizable materials to be emitted from the device and the thermogenic materials which serve to provide the thermal energy to volatilize such materials. Both the volatilizable materials which are held within the volatilization zone and the thermogenic materials which are held within a heating zone may be sealed from the atmosphere surrounding the insert until the device is activated, for example by rupturing the seals covering these zones to thereby expose their contents to the outside air.

### i) Volatilization Zone

One essential component of the devices herein is the volatilization zone. It is the volatilization which holds the volatilizable materials which the dispensing devices of this invention are designed to emit. The volatilization zone can be of any shape or configuration but will generally be substantially rigid.

Preferably the volatilization zone will have a volume of from 1 cm³ to 50 cm³, more preferably from 3 cm³ to 15 cm³. It will also preferably be elongated, having a major dimension of from 1 cm to 15 cm, more preferably from 2 cm to 10 cm. Preferably the volatilization zone will be configured within the device, e.g., within the insert, such that the major dimension of the volatilization zone is generally in a vertical position as the insert is held within the dispensing device.

Frequently the volatilization zone, e.g., within the insert, will be generally cylindrical in configuration. Alternatively, the volatilization zone can have a tapered configuration. The volatilization zone, in any event, must have a discharge end which is open or openable to the surrounding atmosphere. Preferably the volatilization zone will be openable to the atmosphere at both of its ends. European Patent Application No. 00870261.5; Filed November 11, 2000 (P&G Case No.CM-2467F) is directed to devices having a volatilization zone of this configuration

In the cylindrical configuration, the volatilization zone will typically have a circular cross-sectional area which ranges from 1 cm² to 10 cm² more preferably from 2 cm² to 6 cm². In the tapered configuration, the volatilization zone will generally have a smaller circular cross-sectional area at or near the top of the zone than at or near the bottom of the zone. For the tapered configuration, the circular cross-sectional area at or near the top of the volatilization zone will typically range from 0.5 cm² to 5 cm², more preferably from 1 cm² to 3 cm², and the circular cross-sectional area at or near the bottom will typically range from 1 cm² to 10 cm², more preferably from 2 cm² to 6 cm². In the tapered configuration, the volatile or volatilizable materials tend to move through the volatilization zone faster at or near the top by virtue of the effect of the Bernulli principle. This helps to boost the effectiveness of the volatilized material emission into the atmosphere surrounding the device.

The volatilization zone, as with the rest of the elements of the dispensing device herein, can be made of any suitable material which provides the desired rigidity, strength, heat resistance/heat conductivity and compatibility with the materials to be held therein. Typically thermoplastic or thermosetting materials such as polyethylene, polypropylene, polystyrene, acrylic resins, and the like, may be utilized to form the volatilization zone, e.g., within the insert.

The volatilization zone will contain the volatile and/or volatilizable materials which are to be emitted from the device upon rupturing of the volatilization zone sealing means and exposure of this zone to this surrounding atmosphere. Volatilizable materials are those which may be completely or partially in a liquid or solid phase at ambient temperature (20 °C) but which can be vaporized by the heat generated by the thermogenic materials in the heating zone of the devices herein.

Suitable volatilizable materials are those which can act as fragrances, insect repellents, insecticides and materials which are or which produce therapeutic vapors. Preferred volatilizable materials are fragrances which can include any odoriferous materials having a vapor pressure of at least 17.5 mmHg @ 20 °C. Generally, the devices herein will contain from 0.1 gram to 10 grams, more preferably from 0.5 gram to 3 grams, most preferably from 1 gram to 2 grams of the volatilizable materials, generally sealed until activation within the volatilization zone.

The volatilizable materials may be placed into the sealed volatilization chamber in neat form, or they may be held in the volatilization zone in combination with a liquid or solid carrier substrate. Any material which will absorb, adsorb, or otherwise hold or carry the volatilizable material until the volatilization zone is exposed to the atmosphere can be employed as carrier or carrier substrate. Especially suitable carriers can include solid absorbent materials such as felt or cellulosics including paper, cotton, airfelt, and the like. Other carrier include porous polyolefin materials. An especially preferred carrier for materials to be held in the volatilization zone is the porous polypropylene material marketed under the tradename, Accurel™.

### ii) Heating Zone

The volatilization zone of the devices herein is juxtaposed and in thermodynamic communication with at least one heating zone containing air-activatable thermogenic materials. The heating zone, like the volatilization zone, can be constructed of any suitable material which will hold thermogenic materials and be compatible with such materials and the heat generated thereby when the devices herein are activated. Preferably the heating zone will have a volume of 10 cm³ to 100 cm³, more preferably from 20 cm³ to 40 cm³.

In preferred embodiments wherein the volatilization zone is generally cylindrical, the heating zone will be of generally annular configuration, surrounding the volatilization zone, with the generally cylindrical volatilization zone running through the core of the heating zone.

The heating zone will contain thermogenic materials which produce thermal energy via a non-combustive oxidation reaction upon contact with oxygen-containing gas such as for example upon exposure to and contact with air. Typically such materials are those used to bring about the exothermic oxidation of iron and are used in particulate form. Generally the heating zone will contain from 10 grams to 50 grams, more preferably from 15 grams to 30 grams, of the thermogenic material combination.

A preferred combination of air-activatable thermogenic materials comprises from 30% to 80% by weight of the combination of iron powder, from 3% to 25% by weight of the combination of carbon particles, from 0.5% to 10% by weight of the combination of a metal salt such as alkali metal or alkaline earth metal salts, and from 1% to 40% by weight of the combination of water. Such thermogenic material combinations may also contain an additional water-holding particles (e.g., vermiculite, porous silicates) present to the extent of from 0.1% to 30% by weight of the combination. Other optional ingredients in the thermogenic material combination include oxidation reaction enhancers (chromium, manganese, copper), hydrogen gas inhibitors (sodium hydroxide, sodium thiosulfate), fillers (cellulosics), anti-caking agents (tricalcium phosphate, sodium silicoaluminates), thickeners (starches) and surfactants. Thermogenic materials of the type useful in the heating zone of the inserts herein are described in greater detail in Burkett et al; U.S. Patent 5,918,590; Issued July 6, 1999.

The thermogenic materials used in the heating zone should, upon activation, be capable of producing a temperature within the heating zone of at least 60 °C within a period of from 3 to 7 minutes after contact of air with the thermogenic materials is initiated. Preferably, a temperature of at least 50 °C will be maintained for a period of at least 180 minutes, more preferably at least 240 minutes, after activation of the device.

The dispensing devices herein operate most effectively if the devices comprise means for enhancing exposure of the thermogenic materials therein to activating air upon activation of the device. For purposes of this invention, exposure of the thermogenic materials to air is "enhanced" if thermogenic material is contacted with more air than is provided by the simple exposure of the outside surface of the themogenic material when the heating zone seal is ruptured. Thus, for example, air exposure to the thermogenic materials can be enhanced by providing the thermogenic materials in particulate form and packed in a manner such that the mass of thermogenic materials has a porosity which is suitable to permit permeation of air thereinto. This can be accomplished by altering the particle size of the thermogenic materials or by providing structurants or spacers (e.g., starches, glass beads, styrofoam particles, etc.) within the mass of thermogenic material particles. Alternatively, or in addition, air inlet channels such as perforated tubes or screens, can be positioned within and preferably throughout the mass of thermogenic materials.

In another preferred configuration, the thermogenic materials within the heating zone are positioned within at least two distinct regions wherein, upon activation of the device, thermal energy is produced in one such region at a faster rate and for a shorter period of time than thermal energy is produced in at least one of the other such regions. Heating zone configurations of this type are described in greater detail in European Patent Application No. 00870263.1; Filed November 7, 2000 (P&G Case CM-2469F).

The thermogenic materials in the heating zone are preferably held and constrained within this zone by air-permeable holding means. Such holding means serves to prevent the generally solid, e.g., granular, thermogenic materials from being removed from the device, either purposefully or unintentionally, once the sealing means of the heating zone have been ruptured and the heating zone is thereby opened to the outside atmosphere. Such air-permeable holding means, can comprise, for example, fabric- or polymer-based mesh materials having mesh openings, e.g., apertures, therein which are large enough to permit suitable flow of air therethrough but small enough to prevent significant amounts of the solid thermogenic material from passing through the mesh.

### iii) Sealing Means

Both the volatilization zone and the heating zone herein will generally be hermetically sealed from the outside atmosphere by sealing means. Such sealing means serve to prevent the escape of the volatilizable materials from the volatilization zone, and to prevent the activation of the thermogenic materials in the heating zone, until the sealing means are ruptured or otherwise removed. The sealing means also keep the contents of the volatilization zone and the heating zone out of contact with each other. The sealing means can thus comprise part of the means for controlling contact of the thermogenic materials with air. In particular, the sealing means will generally be the aspect of the air contact control means which relates to the initiation of thermal energy produced by the thermogenic materials.

Preferably the sealing means for both volatilization zone and the heating zone will be located at more than one place on each zone. In a preferred configuration for the inserts, wherein both the volatilization and heating zones are elongated and held in a generally vertical position, the rupturable sealing means will be positioned at both the upper and lower end of each zone. In this way, when the sealing means are ruptured or otherwise removed from both ends of these zones, airflow into and through each zone is promoted.

Sealing means can comprise any kinds of materials which are both air and liquid impervious when intact but which can be readily ruptured or otherwise removed when the dispensing device is to be activated. Suitable sealing means for both volatilization and heating zones include any type of single ply or laminated film, foil or sheet which has the desired type of impermeability and rupturability characteristics. Laminated aluminum foil is especially preferred as a sealing means useful in the devices of this invention. The sealing material may, in fact be co-laminated with holding means for the thermogenic material, e.g., with flexible mesh. In this setup, the rupturing means of the holder may be configured to rupture the foil sealing means but not the flexible mesh holding means laminated to the foil.

### B. Holder

The inserts of the preferred device configuration, as described hereinbefore, can be held with the volatilization chamber in a generally upright vertical position by a holder component for the insert. In the preferred devices herein, the holder comprises a substantially rigid base and a substantially rigid cover for the base. The shape of the holder cover and holder base will, of course, depend upon the shape and configuration of the insert which is to fit inside the holder. For inserts which are generally cylindrical in configuration, the base and cover comprising the holder will generally be cylindrical or spherical.

The holder element of the preferred devices herein can have means positioned on the cover or base, and preferably on both, for rupturing the sealing means used for the chambers or zones of the insert. Such rupturing means will generally be consumer activatable, i.e., when the user of the dispensing device wants to activate it, the user will be able to cause the rupturing means to puncture or rupture the sealing means on the insert zones so that exposure to air of the thermogenic materials begins the heat generation process and exposure to air of the volatilizable materials begins the process of dispensing these materials into the surrounding atmosphere. Generally the seal rupturing means associated with the base and/or cover can simply comprise rigid and relatively sharp protrusions which can be forced against the sealing means until rupture of the sealing means occurs.

In one preferred configuration, the covering base of the holder are slideably affixed to each other such as the user of the device can activate it by pushing the cover and base closer together. As illustrated in the drawing hereinafter, this action serves to push the puncturing protrusions which are part of both base and cover into and through the seals of the insert which is held inside the slideably closeable base and cover.

The holder may also preferably be constructed of thermochromic material such that at least a portion of the outside surface of the holder changes color upon heating. In this manner, the device can be constructed of a material which changes color when the device is activated and the holder heats up as a result of thermal energy generation in the heating zone(s). This provides a signal to the user of the device that the device has been activated and is generating heat and emitting volatiles.

### C) Air Contact Control Means

The devices of the present invention essentially comprise some means for controlling the contact of the thermogenic materials within the device with activating air. Such contact control means must include some means for initiating contact of the thermogenic materials with air to begin production of thermal energy within the device. As indicated hereinbefore, such air contact initiating means can comprise the utilization of sealing means which can be ruptured or otherwise removed in order to expose the thermogenic materials to activating air. Other initial air contact means can be provided by the utilization of a holder which can be manipulated to initially expose the thermogenic materials in the heating zone of the device to activating air.

The air contact control means must further include additional means for, after initial contact with activating air has been made, either increasing, decreasing and/or terminating the generation of thermal energy being produced by the thermogenic materials. Such additional means will generally serve to regulate the flow or amount of air being brought into contact with the thermogenic materials. Increasing the flow or amount of air will increase the amount of thermal energy produced. Decreasing the flow or amount of air will decrease the amount of thermal energy produced within the device. Terminating the flow or amount of air will stop the production of thermal energy and will therefore shut the device off.

In one embodiment, the additional air contact control means can be provided by the configuration of the holder element. For example, the cover and base elements of the holder may be configured to control or alter the flow of air into and through the dispensing device once the seals have been ruptured. This can be accomplished by providing and altering the size of air inlet openings and/or the configuration and number of seal puncturing means. The cover and base elements of the holder may also be rotatable or otherwise moveable in relation to each other in order to vary the size of air inlet openings therein.

In another embodiment, the additional air contact control means can comprise a separate airtight container into which the activated dispensing device can be placed. Such a container can itself comprise a base and a cover which can be separated in order to permit insertion of the activated dispensing device and then closed to form an airtight container which surrounds the device. Enclosure of the activated device in the airtight container thus serves to terminate contact of air with the thermogenic materials within the device and thereby cause cessation of thermal energy production within the device. Removal of the dispensing device from the airtight enclosing container will, of course, reactivate the dispensing device.

### D) Optional Volatilization Zone Closure Means

The air contact control means can be used to effectively decrease or shut down emission of volatilized materials for the volatilization zone by reducing or eliminating production of volatilizing thermal energy. However, in preferred devices herein additional closure means will also be provided to restrict or eliminate communication of the volatilization zone itself with the atmosphere surrounding the device, after the device has been activated. Such optional closure means can thus serve to diminish or re-cover (e.g., reseal) the open discharge end of the volatilization zone. As with the air contact control means, the closure means for re-covering or resealing the discharge end of the volatilization zone may be provided by an appropriate configuration of adjustable holes or openings in the holder element used with preferred dispensing devices. Alternatively, placement of the activated device within an airtight container surrounding the device will also have the effect of resealing the volatilization zone from the surrounding atmosphere in addition to serving as the air contact control means.

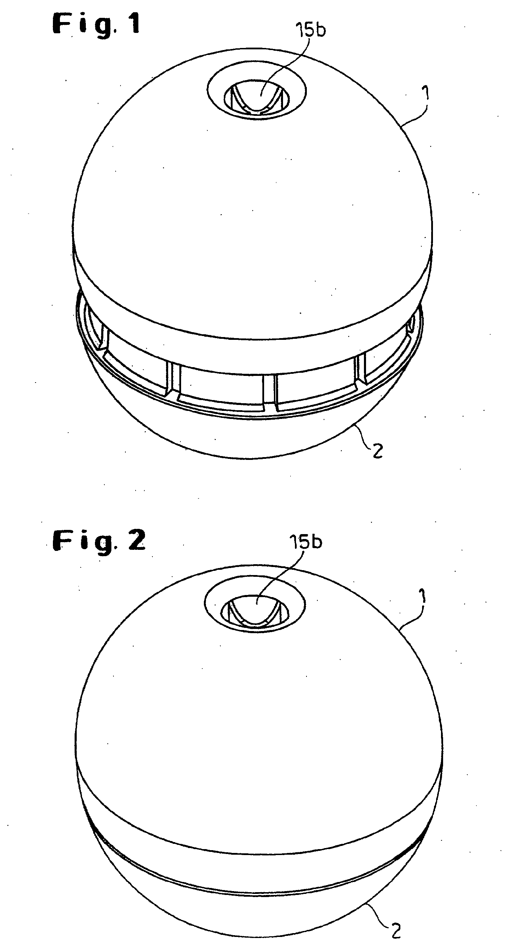

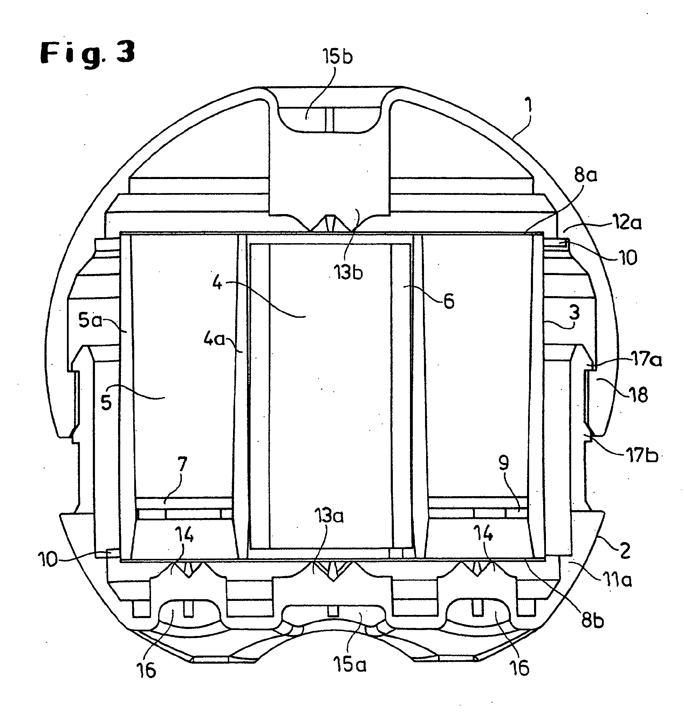

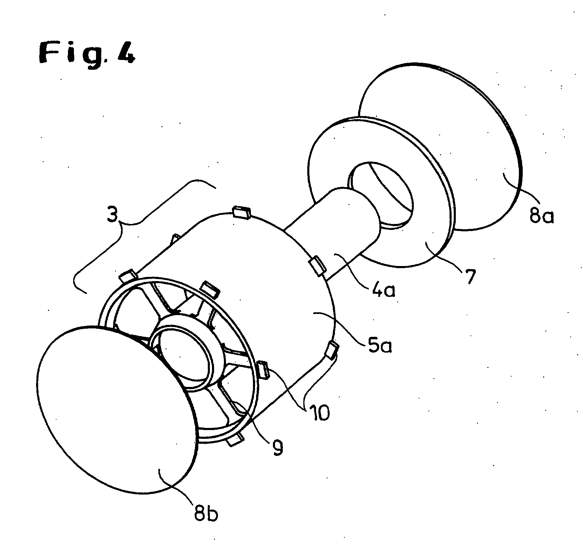

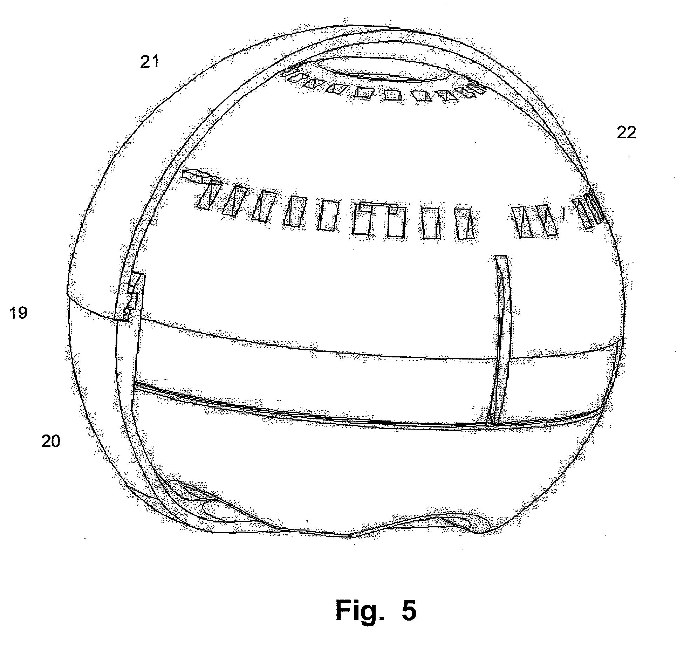

A highly preferred dispensing device is one having a cylindrical insert held within a spherical holder. Such a device, without the essential air contact control means, is depicted in Figs. 1-4 of the drawings. Figure 1 is an external view of the dispensing device in its preactivated condition. Figure 2 is an external view of the dispensing device which has been activated by pushing the holder pieces together. Figure 3 is a cross-sectional view of the device in its preactivated condition. Figure 4 is an exploded view of the insert component of the dispensing device. Figure 5 then shows the dispensing device of Figures 1-4 in combination with the essential air contact control means which in this illustration comprises a surrounding airtight container.

In the several figures there is shown a dispensing device holder comprising a cover **1** which snaps onto a base **2.** Inside the cover and base is a generally cylindrical insert **3.** The insert comprises a cylindrical volatilization zone **4** along the axis of the insert as defined by cylinder wall **4a.** The volatilization zone is surrounded by an annular chamber comprising the heating zone **5** as defined by outside cylindrical wall **5a.** Positioned within the volatilization zone is a carrier tube **6** of Accurel onto which is absorbed an effective amount of a perfume material (not shown). Positioned within the annular heating zone is an effective amount of thermogenic materials (not shown) which are held in place within the heating zone by air permeable wad **7.** Prior to device activation, both the volatilization zone and the heating zone, with the volatile/volatilizable materials and thermogenic materials therein respectively, are sealed at both the top via a top seal **8a** and the bottom via bottom seal **8b.**

The insert is provided with a support spoke **9** which supports the permeable wad **7.** The support spoke has breakable support lugs **10** which rest on the base shoulder **11a** and support the cover at shoulder **16** before the device is activated. The base is provided with a central bottom seal penetrating protrusion **13a** which is positioned to rupture the bottom seal and open the volatilization zone. The base is also provided with four perimeter bottom seal penetrating protrusions **14** positioned to also puncture the bottom seal and open the heating zone. The base is also fitted with a bottom center air inlet opening **15a** and other bottom perimeter air inlets **16** to provide exposure of the volatilization zone and heating zone, respectively, to the atmosphere upon rupture of the bottom seals when the user activates the device.

The cover is also provided with a central top seal penetrating protrusion **13b** and may contain other protrusions (not shown) which are positioned to also puncture the top seal and open the volatilization zone further. The cover also includes a top outlet opening **15b** through which the volatile or volatilized materials are emitted to the outside atmosphere upon activation of the dispensing device. The base is also provided with a clip feature formed by ridges **17a** and **17b** into which snaps the cover clip feature **18.** The clip features keep the cover and base apart, and the seals unpunctured, prior to activation of the device.

After the device depicted in Figures 1-4 has been activated, it can be placed within an airtight container as shown in Figure 5. The container **19** comprises a base **20** and a cover **21** which snap together to form an airtight seal. When the activated dispensing device **22** shown in Figure 2 is placed within the airtight container, air contact with the thermogenic materials within the device is cut off and heat generation within the device ceases.

### Mode of Operation

### Prior to activation:

The insert **3** is suspended inside the holder cover **1** and holder base **2.** The cover and base are kept apart to prevent rupturing of the insert seals **8a** and **8b** by interacting with clip feature **17a, 17b** and **18** and breakable support lugs **10.** A further anti-activation device (not shown in the drawings) could also be used to prevent movement of the cover and base towards each other. Such anti-activation devices could include a clip or shrink sleeve or tamper band.

### To activate:

The cover and base **1** and **2** are pushed together by overcoming clips **17a** and **17b** and **18,** breaking or deforming lugs **10** and piercing seals **8a** and **8b** via the cutter protrusions **13a** and **13b** and **14.** This results in air being allowed to flow through the volatilization zone **4** and the volatile, e.g., fragrance, carrier insert **6** and also into the heating zone **5.** This results an exothermic reaction taking place in the heating zone **5,** which results in heat transfer from the heating zone into the volatilization zone. This, in turn, creates convection currents in the volatilization zone and enhances volatilization of the volatilizable materials which are discharged into the atmosphere through opening **15a.** The clips prevent the cover from being removed from the holder base and ensure that the rupturing protrusions stay in place keeping air channels **15a** and **16** open.

### To Turn Off:

To turn the device off, the device **22** is placed inside the container base **20** and the container cover **21** is then snapped into place over the container base **20.** This serves to place the dispensing device **22** inside the resulting closed container **19.** Shortly thereafter, heat generation within the dispensing device ceases.

## Claims

1. A device suitable for providing controlled discharge of volatilized active materials into the atmosphere surrounding the device, said device being **characterized in that** it comprises:
A) a volatilization zone having a discharge end which is open or openable to the atmosphere;
B) a volatilizable active material placed within said volatilization zone;
C) a heat generation zone in thermodynamic communication with said volatilization zone, said heat generation zone containing thermogenic materials capable of producing thermal energy via a non-combustive thermogenic reaction when contacted with air, said thermogenic materials further being capable of transferring thermal energy so produced to said volatilization zone to thereby volatilize said volatilizable active material and to further permit and promote discharge of said volatilized material through the discharge end of said volatilization zone into the surrounding atmosphere; and
D) means for controlling the contact of said thermogenic materials with air to thereby initiate, and to also thereafter increase, decrease and/or terminate the generation of thermal energy produced by said thermogenic materials via said non-combustive thermogenic reaction.

2. A dispensing device for controlled emission of volatilized materials into the atmosphere surrounding the device, said device being **characterized in that** it comprises:
A) an insert comprising:
i) a substantially rigid volatilization zone containing volatilizable materials, said volatilization zone having an open or openable discharge end and being juxtaposed with and in thermodynamic communication with:
ii) a heating zone containing air-activatable thermogenic materials which produce thermal energy upon contact with air;
B) a holder for said insert, said holder comprising:
i) a substantially rigid base; and
ii) a substantially rigid cover for said base; and
C) means for controlling the contact of said thermogenic materials with air to thereby initiate, and to also thereafter increase, decrease and/or terminate the generation of thermal energy produced by said thermogenic materials.

3. A device according to Claim 1 or Claim 2 wherein said air contact control means comprises hermetic sealing means for both the volatilization zone and the heating zone, said sealing means preventing contact of the contents said zones with each other and with the outside atmosphere prior to activation of said device by the rupture or otherwise removal of said sealing means.

4. A device according to Claim 3 wherein said holder for the insert carries the insert in a manner which maintains the integrity of the sealing means for the zones of the insert until rupturing of said sealing means is desired.

5. A device according to any of Claims 2 to 4 wherein the air contact control means comprises adjustable air inlet holes within said holder.

6. A device according to any of Claims 1 to 5 wherein said air contact control means comprises a separate airtight container into which said dispensing device can be placed.

7. A device according to Claim 6 wherein said airtight container comprises both a base and a cover.

8. A device according to any of Claims 1 to 7 wherein the volatilizable materials within said volatilization zone are selected from the group consisting of fragrances, insect repellents, insecticides and materials which are or which produce therapeutic vapors.

9. A device according to any of Claims 1 to 8 wherein the thermogenic materials within said heating zone are capable of generating thermal energy via an iron oxidation reaction.

10. A device according to Claim 9 wherein said thermogenic materials comprise a combination of iron, carbon, water and inorganic salts.

11. A device according to any of Claims 1 to 10 wherein said thermogenic materials are held or constrained within said heating zone by air-permeable holding means.

12. A device according to any of Claims 1 to 11 wherein said thermogenic materials are capable of producing a temperature within said heating zone of at least 60 ° C within a period of 3 to 7 minutes after contact of air with said thermogenic materials is initiated.

13. A device according to any of Claims 1 to 12 wherein said volatilization zone has a generally vertical major dimension which ranges in length from 1 cm to 15 cm.

14. A device according to Claim 13 wherein said volatilization zone is generally cylindrical.

15. A device according to Claim 13 wherein said volatilization zone is generally of tapered configuration such that its horizontal cross-sectional area at or near the top of its vertical dimension is smaller than its horizontal cross-sectional area at or near the bottom of its vertical dimension.

16. A device according to any of Claims 1 to 15 wherein said volatilization zone has a horizontal cross-sectional area which is generally circular.

17. A device according to Claim 13 wherein said heating zone is of generally annular configuration and completely surrounds said volatilization zone.

18. A device according to any of Claims 1 to 17 which further comprises means for enhancing exposure of said thermogenic materials to air upon initial contact of said thermogenic materials with activating air, which air exposure enhancing means are selected from the group consisting of:
A) utilizing thermogenic materials which are in particulate form and which are packed into a mass having a porosity such that air will permeate into the mass of thermogenic particulate materials:
B) providing at least one air inlet channel within a mass of said thermogenic materials; and
C) combinations of said air exposure enhancing means.

19. A device according to any of Claims 1 to 18 wherein said device additionally comprises volatilization zone closure means which can restrict or eliminate communication of the volatilization zone with the atmosphere surrounding the device after said device has been activated.

20. Use of a dispensing device according to any of Claims 1 to 19 by initiating contact of said thermogenic materials within said device with air and by thereafter increasing, decreasing and/or terminating contact of air with said thermogenic materials.
